# EUROPEAN PATENT APPLICATION

(11) **EP 4 768 057 A1**
(43) Date of publication of application: **01.07.2026**
(21) Application number: 24307334.3
(22) Date of filing: 30.12.2024
(51) Int. Cl.: A61M 5/315

(54) **PLUNGER ROD FOR SYRINGE**

(71) Applicant: Becton Dickinson France, 38800 Le Pont-de-Claix (FR)
(72) Inventor: Lavigne, Ferdinand, 38800 Le Pont-de-Claix (FR); Piloy, Jefferson, 38800 Le Pont-de-Claix (FR); Détroyat, Maël, 38800 Le Pont-de-Claix (FR); Zadorian, Mahis, 38800 Le Pont-de-Claix (FR)
(74) Representative: Germain Maureau

(57) **Abstract**

A plunger rod including an elongated body having a proximal end, a distal end opposite the proximal end, and a longitudinal axis extending between the proximal end and the distal end, wherein in a cross-sectional plane transverse to the longitudinal axis, the elongated body including: a first segment defining a first end and a second end; a second segment laterally spaced from the first segment, the second segment defining a first end and a second end; a third segment extending from the first segment to the second segment, wherein the third segment is axially spaced from the first end of the first segment and the first end of the second segment; a fourth segment extending from the second end of the first segment; and a fifth segment extending from the second end of the second segment.

## Description

### FIELD OF THE INVENTION

The present disclosure relates generally to a plunger rod for use in a syringe barrel which may be associated with an autoinjector, and further relates to a syringe, such as manual syringe, including a plunger rod assembly.

### Background of the invention

Syringes, particularly manually operated hypodermic syringes, are well known in the medical field for dispensing fluids, such as medications, or for withdrawing a biological fluid sample therein. A conventional syringe assembly typically includes a syringe barrel with a fluid opening at one end and a plunger assembly disposed through the opposite end, provided in slideable arrangement through the syringe barrel. The plunger assembly typically includes a rigid plunger rod having a linear longitudinal axis extending through the barrel, with a plunger head or stopper located at a distal end of the plunger rod, and with a thumb press provided at the proximal end of the plunger rod. In use, the plunger rod may be retracted through the syringe barrel to aspirate or fill the syringe barrel with a fluid, such as a medicament. The plunger rod may also be advanceable through the syringe barrel to expel a fluid, such as a medicament, from within the syringe barrel. For delivery of medication to a patient, the opening of the syringe barrel may be adapted for fluid communication with a patient, such as through a hypodermic needle fitted at the distal end of the syringe barrel or through a standard luer-type fitting extending from the distal end of the syringe barrel for attachment with a fluid line. Operation of a plunger rod assembly within a syringe barrel is well known in the medical field.

Numerous syringes are used each year in standard medical settings. As a result, it is desirable that the plunger rods of the syringes be manufactured as cost efficiently as possible, be relatively strong (e.g., in order to withstand loads that are applied during use), and be manufactured with a relatively short cycle time during molding. Accordingly, there is a need in the art for such an improved plunger rod and syringe including the same.

### Summary of the invention

In accordance with one aspect, the present disclosure provides a plunger rod for use with a syringe having a syringe barrel, the plunger rod comprising: an elongated body having a proximal end, a distal end opposite the proximal end, and a longitudinal axis extending between the proximal end and the distal end, wherein in a cross-sectional plane transverse to the longitudinal axis, the elongated body comprises: a first segment defining a first end and a second end; a second segment laterally spaced from the first segment, the second segment defining a first end and a second end; a third segment extending from the first segment to the second segment, wherein the third segment is axially spaced from the first end of the first segment and the first end of the second segment; a fourth segment extending from the second end of the first segment; and a fifth segment extending from the second end of the second segment.

In some non-limiting embodiments or aspects, the first segment and the second segment may each be substantially linear.

In some non-limiting embodiments or aspects, the first segment may be substantially parallel to the second segment.

In some non-limiting embodiments or aspects, the first segment may have a length between 1 mm and 26 mm.

In some non-limiting embodiments or aspects, the first segment may have a thickness between 0.1 mm and 64 mm.

In some non-limiting embodiments or aspects, the elongated body may define a centerline in the cross-sectional plane, and the first segment and the second segment may each define an angle with respect to the centerline between 0.5 degrees and 10 degrees.

In some non-limiting embodiments or aspects, the third segment may be substantially perpendicular to the first segment.

In some non-limiting embodiments or aspects, the third segment may be substantially linear.

In some non-limiting embodiments or aspects, the fourth segment and the fifth segment may each be arcuate.

In some non-limiting embodiments or aspects, the fourth segment and the fifth segment may each comprise an arc length between 0 degrees and 180 degrees.

In some non-limiting embodiments or aspects, the fourth segment and the fifth segment may each comprise a radius of curvature between 0.1 mm and 84 mm.

In some non-limiting embodiments or aspects, the first segment, second segment, third segment, fourth segment, and fifth segment may be contiguous with each other.

In some non-limiting embodiments or aspects, the plunger rod may further comprise one or more support ribs extending from the elongated body between the proximal end and the distal end.

In some non-limiting embodiments or aspects, the plunger rod may further comprise a stopper coupled to the distal end of the elongated body.

According to non-limiting embodiments or aspects, a syringe assembly is provided, comprising: a syringe barrel having a proximal end, a distal end, and an interior cavity; a stopper moveably positioned within the interior cavity of the syringe barrel; and the plunger rod disclosed herein attached to the stopper.

Further examples of the present disclosure will now be described in the following numbered clauses.

Clause 1: A plunger rod for use with a syringe having a syringe barrel, the plunger rod comprising: an elongated body having a proximal end, a distal end opposite the proximal end, and a longitudinal axis extending between the proximal end and the distal end, wherein in a cross-sectional plane transverse to the longitudinal axis, the elongated body comprises: a first segment defining a first end and a second end; a second segment laterally spaced from the first segment, the second segment defining a first end and a second end; a third segment extending from the first segment to the second segment, wherein the third segment is axially spaced from the first end of the first segment and the first end of the second segment; a fourth segment extending from the second end of the first segment; and a fifth segment extending from the second end of the second segment.

Clause 2: The plunger rod of clause 1, wherein the first segment and the second segment are each substantially linear.

Clause 3: The plunger rod of clause 1 or clause 2, wherein the first segment is substantially parallel to the second segment.

Clause 4: The plunger rod of any of clauses 1-3, wherein the first segment has a length between 1 mm and 26 mm.

Clause 5: The plunger rod of any of clauses 1-4, wherein the first segment has a thickness between 0.1 mm and 64 mm

Clause 6: The plunger rod of any of clauses 1-5, wherein the elongated body defines a centerline in the cross-sectional plane, and wherein the first segment and the second segment each define an angle with respect to the centerline between 0.5 degrees and 10 degrees.

Clause 7: The plunger rod of any of clauses 1-6, wherein the third segment is substantially perpendicular to the first segment.

Clause 8: The plunger rod of any of clauses 1-7, wherein the third segment is substantially linear.

Clause 9: The plunger rod of any of clauses 1-8, wherein the fourth segment and the fifth segment are each arcuate.

Clause 10: The plunger rod of any of clauses 1-9, wherein the fourth segment and the fifth segment each comprise an arc length between 0 degrees and 180 degrees.

Clause 11: The plunger rod of any of clauses 1-10, wherein the fourth segment and the fifth segment each comprise a radius of curvature between 0.1 mm and 84 mm.

Clause 12: The plunger rod of any of clauses 1-11, wherein the first segment, second segment, third segment, fourth segment, and fifth segment are contiguous with each other.

Clause 13: The plunger rod of any of clauses 1-12, wherein the plunger rod further comprises one or more support ribs extending from the elongated body between the proximal end and the distal end.

Clause 14: The plunger rod of any of clauses 1-13, further comprising a stopper coupled to the distal end of the elongated body.

Clause 15: A syringe assembly comprising: a syringe barrel having a proximal end, a distal end, and an interior cavity; a stopper moveably positioned within the interior cavity of the syringe barrel; and the plunger rod of any of clauses 1-13, wherein the plunger rod is attached to the stopper.

### Brief description of the drawings

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
Fig. 1 is a perspective view of an example of a syringe assembly constructed in accordance with the principles of the present invention.
Fig. 2 is a cross-sectional view of the syringe assembly of Fig. 1.
Fig. 3 is a perspective view of an example of a plunger rod constructed in accordance with the principles of the present invention.
Fig. 4 is a perspective cross-sectional view of the plunger rod of Fig. 3.
Fig. 5 is a cross-sectional view of the plunger rod of Fig. 3.

### Description of the invention

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the concept as it is oriented in the drawing figures. As used herein, the distal end of a component or of a device is to be understood as meaning the end furthest from the user's hand, and the proximal end is to be understood as meaning the end closest to the user's hand. However, it is to be understood that the concept may assume various alternative variations, except where expressly specified to the contrary. It is also to be understood that the specific devices illustrated in the attached drawings, and described in the following specification, are simply exemplary embodiments of the concept. Hence, specific dimensions and other physical characteristics related to the embodiments disclosed herein are not to be considered as limiting.

The word "comprising" and "comprises", and the like, does not exclude the presence of elements or steps other than those listed in any claim or the specification as a whole. In the present specification, "comprises" means "includes" and "comprising" means "including."

As used herein, "at least one of" is synonymous with "one or more of." For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The term "at least" is synonymous with "greater than or equal to." The terms "first", "second", and the like are not intended to refer to any particular order or chronology, but refer to different conditions, properties, or elements. As used herein, the singular form of "a", "an", and "the" include plural referents unless the context clearly dictates otherwise.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

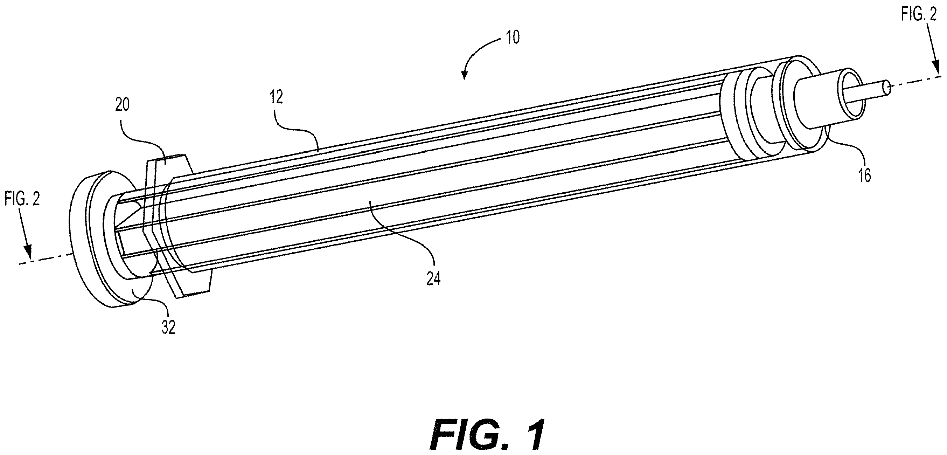

Reference is now made to Figs. 1-2, which show a syringe assembly, generally indicated as 10. The syringe assembly 10 may include a syringe barrel 12 with a proximal end 14, a distal end 16, and an interior cavity 18. A flange 20 may extend radially outward from the proximal end 14 to facilitate grasping and operation of the syringe assembly 10. The syringe barrel 12 may be in the general form of an elongated cylindrical barrel as is known in the art in the general shape of a hypodermic syringe. In alternative examples, the syringe barrel 12 may be in other forms for containing a fluid for delivery. The syringe barrel 12 may be formed of glass, may be injection molded from thermoplastic material such as polypropylene and polyethylene, or may be made from other suitable materials and according to other applicable techniques.

The syringe assembly 10 may include a stopper 22 movably positioned within the interior cavity 18 of the barrel 12, and configured to move fluid in and/or out of the interior cavity 18 of the barrel 12. The stopper 22 may be sized relative to syringe barrel 12 to provide a sealing engagement with the interior surface of the interior cavity 18 of the syringe barrel 12. The stopper 22 may be constructed from one or more polymeric materials such as polyurethane (PU), silicon, and/or a thermoplastic elastomer (TPE).

The syringe assembly 10 may include a plunger rod 24 coupled to the stopper 22 and configured to move the stopper 22 within the barrel 12 to operate the syringe assembly. The syringe assembly 10 may be adapted for the dispensing and delivery of a fluid and/or collection of a fluid. For example, the syringe assembly 10 may be used for injection or infusion of fluid such as a medication into a patient. The syringe assembly 10 may be adapted for aspirating a fluid into the syringe barrel 12, such as withdrawing a medication into the syringe interior and/or withdrawing a fluid sample therein. The syringe assembly 10 is contemplated for use in connection with a patient needle, such as by connecting the syringe assembly 10 to a separate needle assembly (not shown), or alternatively for connection with an intravenous (IV) connection assembly (not shown). The present disclosure provides features that can be used with any type of syringe, including those which are placed in a controlled storage environment in which storage space is limited. These types of syringes may include traditional pre-filled syringe assemblies, metered dose syringes, aspiration syringes for withdrawing fluid from a patient or medication from a container, and the like.

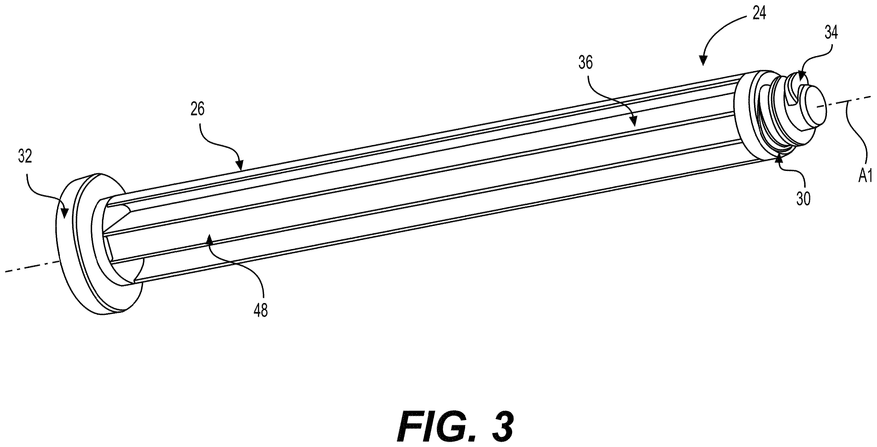

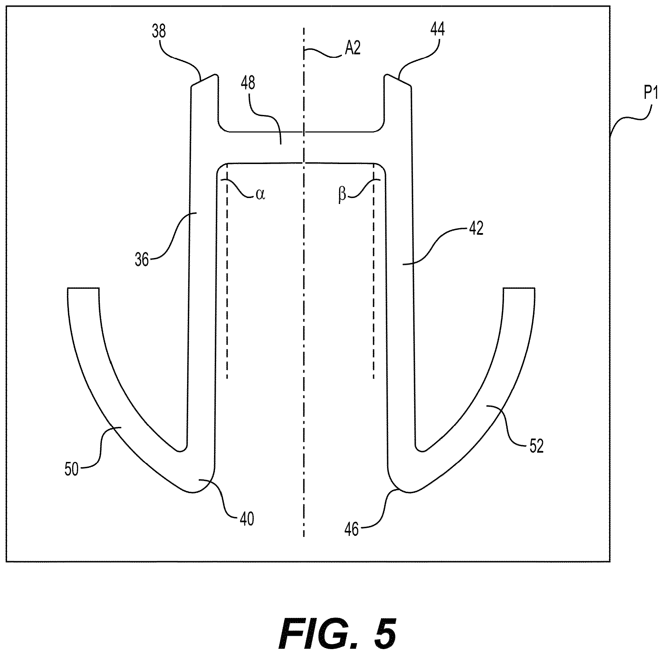

Referring now to Figs. 3-5, the plunger rod 24 may generally include or define an elongated body 26 with a proximal end 28, a distal end 30, and a longitudinal axis A1 extending from the proximal end 28 to the distal end 30. The proximal end 28 may include or define a thumb press 32 configured to present a depressible surface facilitating operation of the plunger rod 24. In one example, the thumb press 32 may include or define a substantial planar surface transverse to the longitudinal axis A1. The thumb press 32 may have a diameter or width greater than a diameter or width of the interior cavity 18 of the syringe barrel 12.

The distal end 30 may include or define an engagement feature 34 configured or adapted to connect or attach to the stopper 22. The engagement feature 34 may include, for example, a threaded surface, one or more clips, protrusions, snaps or the like, and/or alternative connection mechanisms to attach the plunger rod 24 to the stopper 22.

The elongated body 26 of the plunger rod 24 may include improved geometry enabling the plunger rod 24 to be manufactured with less material, and molded with a smaller cycle time while maintaining and/or improving structural integrity and flexibility features, as disclosed further herein. For example, in a cross-sectional plane P1 substantially perpendicular and/or transverse to the longitudinal axis A1, the elongated body 26 may include a plurality of connected, continuous, and/or integral segments. The elongated body 26 may include a first segment 36 with a first end 38 and a second end 40. In one example, the first segment 36 may be substantially linear. In one aspect or example, the first segment 36 may have a length less than an inner diameter of the barrel 12 of the syringe assembly 12. In one aspect or example, the first segment 36 may have a length between 1 mm and 26 mm. In one aspect or example, the first segment 36 may have a length of approximately 5.7 mm for use with a syringe barrel configured to contain or define a volume between 1 mL and 3 mL.

In one aspect or example, the first segment 36 may have a thickness between 0.1 mm and 64 mm. In one aspect or example, the first segment 36 may have a thickness of approximately 0.5 mm for use with a syringe barrel configured to contain or define a volume between 1 mL and 3 mL.

In one aspect or example, the first segment 36 may be angled with respect to a centerline axis A2 extending along the plane P1. The first segment 36 may form an angle α between 0.5 degrees and 10 degrees with respect to the centerline axis A2. In one aspect or example, the first segment 36 may form an angle α of approximately 1 degree with respect to the centerline axis A2 for use with a syringe barrel configured to contain or define a volume between 1 mL and 3 mL.

Continuing to refer to the features in the cross-sectional plane P1, the elongated body 26 may include a second segment 42 with a first end 44 and a second end 46. The second segment 42 may be laterally spaced (e.g., in a direction substantially transverse to the centerline axis A2) from the first segment 36. In one example, the second segment 42 may be laterally spaced from the first segment 36 between 0.1 mm and 30 mm. In one aspect or example, the second segment 42 may be laterally spaced from the first segment 36 by approximately 3 mm for use with a syringe barrel configured to contain or define a volume between 1 mL and 3 mL.

In one example, the second segment 42 may be substantially linear. In one aspect or example, the second segment 42 may have a length between 1 mm and 30 mm, and/or a thickness between 0.1 mm and 64 mm. In one aspect or example, the second segment 42 may be angled with respect to the centerline axis A2 extending along the plane P1. The second segment 42 may form an angle β between 0.5 degrees and 10 degrees with respect to the centerline axis A2. In one aspect or example, the second segment 42 may form an angle β of approximately 1 degree with respect to the centerline axis A2 for use with a syringe barrel configured to contain or define a volume between 1 mL and 3 mL.

Continuing to refer to the features in the cross-sectional plane P1, the elongated body 26 may include a third segment 48 extending from the first segment 36 to the second segment 42. The third segment 48 may be axially spaced (e.g., in a direction substantially parallel to the centerline axis A2) from the first end 38 of the first segment 36 and the first end 44 of the second segment 42. The third segment 48 may be axially positioned closer to the first end 38 of the first segment 36 and the first end 44 of the second segment 42 than to the second end 40 of the first segment 36 and the second end 46 of the second segment 42. In one example, the third segment 48 may be substantially linear. In one aspect or example, the third segment 48 may be substantially perpendicular to the centerline axis A2

Continuing to refer to the features in the cross-sectional plane P1, the elongated body 26 may include a fourth segment 50 extending from the second end 40 of the first segment 36. The elongated body 26 may also include a fifth segment 52 extending from the second end 46 of the second segment 42. The fourth segment 50 and the fifth segment 52 may each be substantially arcuate or curved. In one example, the fourth segment 50 and the fifth segment 52 may each extend laterally outward away from the centerline axis A2. In another aspect, the fourth segment 50 and the fifth segment 52 may each include or define a radius of curvature between 0.1 mm and 84mm. In one aspect or example, the fourth segment 50 and the fifth segment 52 may each include or define a radius of curvature of approximately 0.5 mm for use with a syringe barrel configured to contain or define a volume between 1 mL and 3 mL.

In another aspect or example, the fourth segment 50 and the fifth segment 52 may each include or define an arc length between 0 degrees and 180 degrees. In one aspect or example, the fourth segment 50 and the fifth segment 52 may each include or define an arc length of approximately 90 degrees for use with a syringe barrel configured to contain or define a volume between 1 mL and 3 mL.

In one aspect or example, the fourth segment 50 and the fifth segment 52 may each be substantially linear. One or more of the segments disclosed herein may define or include a substantially flat, planar segment in the longitudinal direction adjacent and/or close to the second end 40 and/or the second end 46 for simplified ejector pin constructions or other manufacturing considerations as described herein.

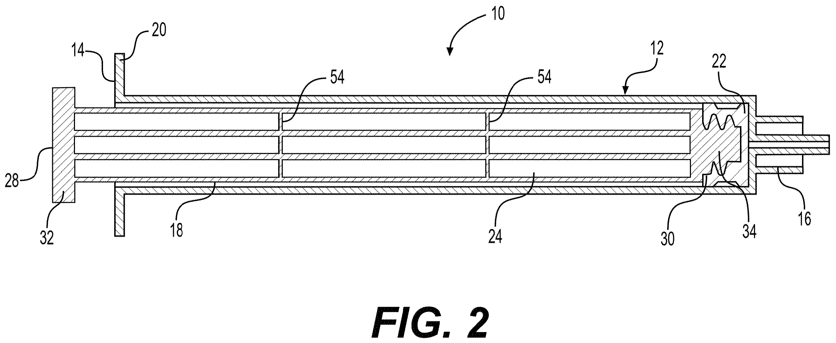

In one example, as shown in Fig. 2, the plunger rod 24 may include one or more support ribs 54 configured or adapted to strengthen and stabilize the plunger rod 24, thereby allowing movement into and out of syringe barrel 4 without significant and undesirable flexion. The one or more support ribs 54 may extend between the fourth segment 50 and the first segment 36 and/or extend between the fifth segment 52 and the second segment 42. The support ribs 54 may extend radially (e.g., orthogonally with respect to the longitudinal axis A1). In one example, a plurality of the support ribs 54 may be spaced along the longitudinal axis and length of the elongated body 26.

In operation, the geometry of the plunger rod provides a number of advantages over known plunger rods. The improved shape maintains the functional attributes necessary to performing the plunging operations, but advantageously allows for a significant reduction in material costs, and cycle time. For example, by having a discontinuous arcuate outer perimeter, and having sections that do not intersect each other, the elongated body can advantageously be manufactured using less material than a conventional plunger rod. In addition, the features of the plunger rod disclosed herein resist breakage with better resistance to flexion (e.g., displacement away from the longitudinal axis) during movement within the syringe barrel. Due to the increased resistance to breakage, plunger rods as disclosed herein may be manufactured out of different, non-traditional materials (e.g., without limitation, thermoplastic material such as a polycarbonate material, polypropylene, polystyrene, etc.).

Moreover, cycle times associated with manufacturing the plunger rod are advantageously reduced due to the elimination of relatively thick regions that might otherwise require long periods of time to cool during molding. As noted above, although less material is being used in the described plunger rod, structural and functional integrity are maintained due to the improved geometry. The improved shape maintains the functional attributes necessary to performing the plunging operations, but advantageously allows for a significant reduction in material costs, and cycle time.

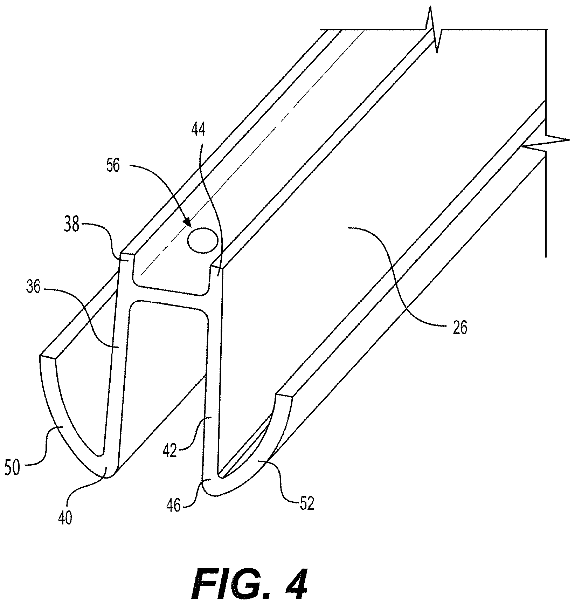

In addition, to manufacture the plunger rod, an injection gate may be positioned on a non-functional surface of the elongated body 26, such as one or more of the surfaces of the first segment, second segment, and/or third segment that do not contact the interior of the syringe barrel 12 during operation of the syringe assembly 10. For example, as shown in Fig. 4, an injection gate may be positioned on or about a location 56 to feed a surface of the third segment 48, which is recessed from the barrel contacting surfaces of the first ends 38, 44 of the first and second segments 36, 42. As such, any residual manufacturing strings, flashes, or ejection marks do not negatively affect the operation of the plunger rod 24.

The features of the plunger rod also allow for a simplified mold design, simplified tooling with an open gate hot-runner system, increased mold cavitation while keeping the same mold size, improved plastic flow to the end-of-fill using a flow line, improved cavity balance with a flooded hot-runner system, simplified part ejection with a stripper block and flat top ejector pin/blade, and reduced ejection force requirements by reduction the core engagement in the part.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A plunger rod for use with a syringe having a syringe barrel, the plunger rod comprising:
an elongated body having a proximal end, a distal end opposite the proximal end, and a longitudinal axis extending between the proximal end and the distal end, wherein in a cross-sectional plane transverse to the longitudinal axis, the elongated body comprises:
a first segment defining a first end and a second end;
a second segment laterally spaced from the first segment, the second segment defining a first end and a second end;
a third segment extending from the first segment to the second segment, wherein the third segment is axially spaced from the first end of the first segment and the first end of the second segment;
a fourth segment extending from the second end of the first segment; and
a fifth segment extending from the second end of the second segment.

2. The plunger rod of claim 1, wherein the first segment and the second segment are each substantially linear.

3. The plunger rod of claim 1, wherein the first segment is substantially parallel to the second segment.

4. The plunger rod of claim 1, wherein the first segment has a length between 1 mm and 26 mm.

5. The plunger rod of claim 1, wherein the first segment has a thickness between 0.1 mm and 64 mm

6. The plunger rod of claim 1, wherein the elongated body defines a centerline in the cross-sectional plane, and wherein the first segment and the second segment each define an angle with respect to the centerline between 0.5 degrees and 10 degrees.

7. The plunger rod of claim 1, wherein the third segment is substantially perpendicular to the first segment.

8. The plunger rod of claim 1, wherein the third segment is substantially linear.

9. The plunger rod of claim 1, wherein the fourth segment and the fifth segment are each arcuate.

10. The plunger rod of claim 1, wherein the fourth segment and the fifth segment each comprise an arc length between 0 degrees and 180 degrees.

11. The plunger rod of claim 1, wherein the fourth segment and the fifth segment each comprise a radius of curvature between 0.1 mm and 84 mm.

12. The plunger rod of claim 1, wherein the first segment, second segment, third segment, fourth segment, and fifth segment are contiguous with each other.

13. The plunger rod of claim 1, wherein the plunger rod further comprises one or more support ribs extending from the elongated body between the proximal end and the distal end.

14. The plunger rod of claim 1, further comprising a stopper coupled to the distal end of the elongated body.

15. A syringe assembly comprising:
a syringe barrel having a proximal end, a distal end, and an interior cavity;
a stopper moveably positioned within the interior cavity of the syringe barrel; and
the plunger rod of any of claims 1-13, wherein the plunger rod is attached to the stopper.
